# FASCICULE DE BREVET EUROPEEN

(11) **EP 4 153 028 B1**
(45) Date de publication et mention de la délivrance du brevet: **11.02.2026**
(21) Numéro de dépôt: 22721084.6
(22) Date de dépôt: 07.04.2022
(51) Int. Cl.: A61B 3/113

(54) **CÔNE DE VISION, DISPOSITIF ET MÉTHODE DE DETECTION DES MOUVEMENTS DES YEUX D'UN PATIENT**
SEHKEGEL, VORRICHTUNG UND VERFAHREN ZUM ERFASSEN DER AUGENBEWEGUNGEN EINES PATIENTEN
VISION CONE, DEVICE AND METHOD FOR DETECTING EYE MOVEMENTS OF A PATIENT

(30) Priorité: 08.04.2021 BE 202105272
(43) Date de publication de la demande: 29.03.2023
(73) Titulaire: P³Lab, 1341 Ceroux-Mousty (BE)
(72) Inventeur: DAYE, Pierre Martin Jack Gérard, 1420 Braine-l'alleud (BE); POUPPEZ DE KETTENIS DE HOLLAEKEN, Antoine Hubert Henri Paul Marie, 1341 Céroux-Mousty (BE)
(74) Mandataire: Gevers Patents
(86) Numéro de dépôt international: PCT/EP2022/059299
(87) Numéro de publication internationale: WO 2022/214609

(56) Documents cités:
- EP-A1- 3 257 435
- WO-A1-2021/022028
- CN-A- 107 205 146
- CN-U- 211 723 123
- KR-A- 20200 107 395
- KR-B1- 102 175 434
- US-A1- 2015 253 574
- US-A1- 2020 209 628
- US-A1- 2020 281 455

## Description

### Domaine technique

La présente invention concerne un cône de vision, un dispositif de détection des mouvements des yeux d'un patient comprenant le cône de vision et une méthode de détection des mouvements des yeux d'un patient mettant en œuvre le dispositif.

### Art antérieur

L'invention se rapporte au domaine de l'identification de troubles oculaires et/ou de troubles neurologiques ainsi qu'à la rééducation des troubles de la vision au travers de l'analyse du mouvement des yeux d'un patient.

Le document US8951046 décrit un agencement pour des tests cognitifs qui élimine les effets environnementaux. L'agencement comprend une enceinte à poser sur une table ayant un masque facial sur une paroi et abritant un écran d'ordinateur sur la paroi opposée. L'agencement permet une mesure de la direction du regard faite en déterminant la position de la pupille lorsqu'un patient presse son visage contre le masque pour éliminer le mouvement de la tête par rapport à l'écran d'ordinateur.

Le document US9004687 décrit un appareil de tests neuropsychologiques impliquant un suivi oculaire. L'appareil comprend un casque adapté à être monté sur la tête d'un patient, le casque ayant un écran positionné à une distance prédéterminée des yeux du patient. Le casque comprend en outre des prismes positionnés entre les yeux du patient et l'écran de telle sorte que les prismes dirigent la lumière émise depuis un point sur l'écran vers les yeux du patient.

L'inconvénient de ces dispositifs est qu'il n'y a qu'une distance possible du visage par rapport à l'écran d'ordinateur ce qui ne permet pas de faire des séries de mesures dans différentes conditions d'éloignement du visage par rapport à des stimuli visuels sur les écrans.

Il y a un besoin pour un appareil qui permette de faire des séries de mesures dans différentes conditions d'éloignement du visage par rapport à des stimuli visuels.

Le document KR20200107395A divulgue (mode de réalisation du figure 103) un cône de vision pour la détection du mouvement des yeux d'un patient, le cône de vision comprenant : une chambre avec une première extrémité et une deuxième extrémité, une paroi latérale délimitant la chambre entre la première extrémité et la deuxième extrémité, un pourtour d'une ouverture apte à être en contact du front du patient à la première extrémité, une interface de fixation d'une unité de calcul à la deuxième extrémité, une première entretoise supportant le pourtour de l'ouverture et une pluralité d'entretoises supplémentaires assemblées les unes aux autres formant la paroi latérale, dans lequel la distance entre le pourtour de l'ouverture et l'interface de fixation est réglable à une longueur fixe par lesdites entretoises supplémentaires formant la paroi latérale.

### Exposé de l'invention

A cet effet, l'invention propose un cône de vision avec les caractéristiques de la revendication 1.

Selon une variante, la dernière entretoise supporte l'interface de fixation de l'unité de calcul à la deuxième extrémité.

Selon une variante, les entretoises forment entre 5 et 15 cm de paroi latérale.

Selon une variante, le cône de vision comprend en outre un ou des orifices traversants dans la paroi latérale, les orifices étant adaptés à l'envoi de stimuli visuels en différentes position dans la chambre.

Selon une variante, les orifices sont alignés entre la première extrémité et la deuxième extrémité.

Selon une variante, le cône de vision est portable sur le visage du patient.

Selon une variante, le cône de vision comprend des organes de fixation du cône à la tête du patient et/ou des poignées.

Selon une variante, le cône de vision comprend un cloisonnement de la vision de chaque œil et/ou un obturateur de la vision d'un des yeux.

Selon une variante, l'obturateur comprend un écran amovible obturant sélectivement la vision d'un œil ou l'autre.

Selon une variante, le cône de vision comprend un filtre devant chaque œil pour sélectionner une partie différente sur chaque œil du stimulus visuel présenté par l'unité de calcul.

Selon une variante, le pourtour de l'ouverture est adaptable au contour du visage du patient et/ou au contour des yeux et/ou à la présence de lunettes sur le visage du patient.

Selon une variante, le cône de vision comprend en outre un cache amovible adapté à obturer l'ouverture, le cache comprenant un motif de calibration de caméra sur sa face tournée vers l'intérieur de la chambre.

Selon une variante, le motif est concave.

Selon une variante, le cône de vision est un ensemble passif.

L'invention se rapporte aussi à un dispositif de détection du mouvement des yeux d'un patient comprenant
- le cône de vision tel que décrit précédemment et
- une unité de calcul fixée à l'interface de fixation du cône de vision, l'unité de calcul comprenant des caméras de suivi du mouvement des yeux et un écran d'affichage de stimuli visuels.

Selon une variante, le cône de vision comprend un cache amovible adapté à obturer l'ouverture, le cache comprenant un motif de calibration des caméras sur sa face tournée vers l'intérieur de la chambre.

Selon une variante, l'unité de calcul comprend les émetteurs infrarouges illuminant la chambre.

Selon une variante, le dispositif comprend en outre un support supportant l'unité de calcul, le cône de vision étant fixé à l'unité de calcul par l'interface de fixation.

L'invention se rapporte aussi à une méthode de détection du mouvement des yeux d'un patient, comprenant les étapes de la revendication 12.

Selon une variante, la méthode comprend en outre une étape de calibration des caméras au cours de laquelle l'ouverture est obturée par un cache amovible comprenant un motif de calibration de caméra sur sa face tournée vers l'intérieur de la chambre.

Selon une variante, le motif de calibration est concave.

Selon une variante, la méthode comprend en outre une étape de stimulation des yeux par l'envoi de stimuli visuels dans la chambre par des orifices traversants dans la paroi.

L'usage, dans ce document, du verbe « comprendre », de ses variantes, ainsi que ses conjugaisons, ne peut en aucune façon exclure la présence d'éléments autres que ceux mentionnés. L'usage, dans ce document, de l'article indéfini « un », « une », ou de l'article défini « le », « la » ou « l' », pour introduire un élément n'exclut pas la présence d'une pluralité de ces éléments.

Les termes « premier », « deuxième », « troisième », etc. sont, quant à eux, utilisés dans le cadre de ce document exclusivement pour différencier différents éléments, et ce sans impliquer d'ordre entre ces éléments.

L'ensemble des modes de réalisation préférés ainsi que l'ensemble des avantages du cône de vision selon l'invention se transposent mutatis mutandis au présent dispositif et au procédé de détection du mouvement des yeux d'un patient.

### Brève description des figures

D'autres caractéristiques et avantages de la présente invention apparaîtront à la lecture de la description détaillée qui suit pour la compréhension de laquelle on se reportera aux figures annexées qui montrent :
- la figure 1, une vue schématique d'un exemple de réalisation du cône de vision selon une vue arrière ;
- la figure 2, une vue schématique d'un exemple de réalisation du cône de vision selon une vue de devant ;
- la figure 3, une vue schématique d'un cache selon un exemple de réalisation ;
- la figure 4, une vue schématique d'un exemple de réalisation du cône de vision selon une vue arrière avec le cache ;
- la figure 5, une vue schématique d'un exemple de réalisation du cône de vision selon une vue de profil ;
- la figure 6, une vue schématique d'un exemple de réalisation de l'unité de calcul.

Les dessins des figures ne sont pas à l'échelle. Des éléments semblables sont en général dénotés par des références semblables dans les figures. Dans le cadre du présent document, les éléments identiques ou analogues peuvent porter les mêmes références. En outre, la présence de numéros ou lettres de référence aux dessins ne peut être considérée comme limitative, y compris lorsque ces numéros ou lettres sont indiqués dans les revendications.

### Description détaillée de modes de réalisation de l'invention

L'invention se rapporte à un cône de vision pour la détection du mouvement des yeux d'un patient. Le cône de vision comprend une chambre avec une première extrémité et une deuxième extrémité, une paroi latérale délimitant la chambre entre la première extrémité et la deuxième extrémité. Un pourtour d'une ouverture apte à être en contact du front du patient est à la première extrémité et une interface de fixation d'une unité de calcul est à la deuxième extrémité. La distance entre le pourtour de l'ouverture et l'interface de fixation est réglable à une longueur fixe. Ainsi, le cône de vision permet de faire des séries de mesures dans différentes conditions d'éloignement du visage par rapport à des stimuli visuels.

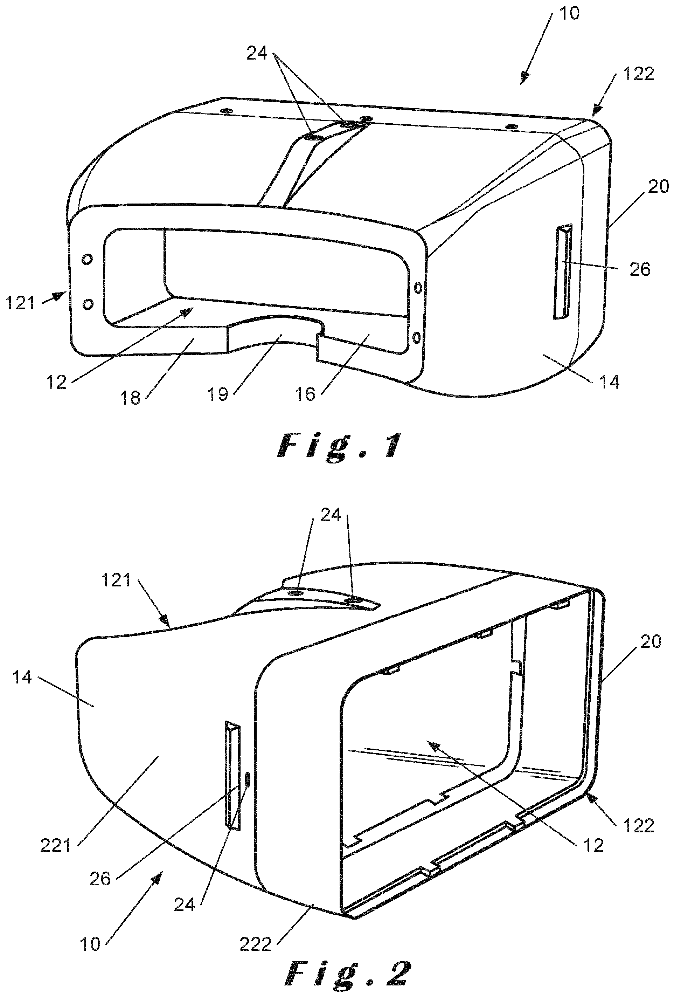

La figure 1 illustre une vue de derrière du cône de vision 10 selon un exemple de réalisation de l'invention. Le cône de vision 10 comprend une chambre 12 avec une première extrémité 121 et une deuxième extrémité 122. La chambre est délimitée par une paroi latérale 14 entre la première extrémité 121 et la deuxième extrémité 122. A la première extrémité 121, le cône de vision 10 comprend une ouverture 16 permettant à un patient de voir à l'intérieur de la chambre 12 et plus spécifiquement comme cela sera décrit par la suite, de voir une unité de calcul à la deuxième extrémité 122. L'ouverture 16 est circonscrite par un pourtour 18 apte à être en contact du front du patient. En d'autres termes, le patient applique le haut du pourtour 18 de l'ouverture 16 contre son front de sorte à avoir son regard pénétrant dans la chambre 12 au travers de l'ouverture 16. Le pourtour 18 de l'ouverture 16 peut être plan mais est de préférence concave vers l'intérieur de la chambre 12 ; ceci permet de bien appliquer le pourtour 18 sur le visage du patient de sorte que le cône 10 bloque l'entrée de la lumière par le côté des yeux afin de rendre l'intérieur de la chambre bien sombre. Le pourtour 18 peut aussi comprendre une cavité 19 pour adapter le pourtour 18 au nez du patient. A la deuxième extrémité 122 de la chambre, le cône 10 comporte une interface de fixation 20 d'une unité de calcul non visible sur la figure 1. Ainsi, une fois le pourtour 18 appliqué contre le front du patient et entourant son champ de vision, le patient peut voir au travers de l'ouverture 16 l'unité de calcul fixé à l'interface de fixation 20.

Afin de faire des séries de mesures dans différentes conditions d'éloignement du visage par rapport à des stimuli visuels diffusés par l'unité de calcul, la distance entre le pourtour 18 de l'ouverture 16 et l'interface de fixation 20 est réglable à une longueur fixe. Autrement dit, la longueur de la paroi latérale 14 délimitant la chambre 12 entre les deux extrémités 121, 122 est ajustable à une valeur souhaitée puis maintenue pour une série de mesures. On règle l'éloignement entre le visage et l'unité de calcul au préalable et le cône de vision maintient cet éloignement durant une série de mesure. Le visage est maintenu éloigné à une distance prédéterminée pour les mesures. Ainsi, la profondeur de la chambre 12 depuis la première extrémité 121 en direction de l'autre extrémité 122 est réglable en faisant varier la longueur de la paroi latérale 14 ; la profondeur choisie est ensuite conservée pour la série de mesures. Le fait que la longueur soit fixe permet ensuite de travailler pour la calibration et durant les séries de mesures dans un environnement normalisé, prédéterminé, aux dimensionnements connues. Cet environnement est pris en compte dans la calibration et tests. L'environnement est normalisé dans le temps et même d'un patient à un autre.

La longueur fixe de la distance entre le pourtour et l'interface de fixation est la longueur entre un point du pourtour et sa projection sur l'interface de fixation, selon la direction du regard du patient. Une longueur de référence de la distance entre le pourtour 18 de l'ouverture 16 et l'interface de fixation est par exemple celle prise au centre du pourtour 18, dans la zone du pourtour 18 qui s'applique au milieu du front du patient, au droit de son nez. Le fait que la longueur soit fixe permet de conserver une certaine distance fixe, prédéterminée, entre le pourtour et l'interface sans modification possible durant un test. Par exemple, le cône permet de rester dans son état malgré un geste brusque du patient (ce qui peut être le cas avec un enfant).

La distance entre le pourtour 18 de l'ouverture 16 et l'interface de fixation 20 peut être réglable à une longueur fixe par une ou plusieurs entretoises formant la paroi latérale 14. Une entretoise est une pièce rigide qui en relie deux autres et les maintient dans un écartement fixe. Le choix de la ou des entretoises permet le réglage de la distance à une longueur fixe, et ce de manière aisée et précise. Les entretoises assurent un écartement figé, constant. Les entretoises ont une longueur fixe (selon la direction du regard du patient). Les entretoises ont une taille (ou longueur, profondeur) normalisée, permettant le réglage de la distance à une longueur fixe. Les entretoises ont une taille (ou longueur, profondeur) qui est connue, prédéterminée, permettant le réglage de la distance à une longueur fixe. L'assemblage d'une ou plusieurs entretoises permet le réglage de la distance à une longueur fixe. L'ajout d'une ou plusieurs entretoises permet le réglage de la distance à une longueur fixe. L'intercalage d'une ou plusieurs entretoises permet le réglage de la distance à une longueur fixe. Le nombre d'entretoises nécessaires est ajusté à la longueur souhaité, ce qui évite d'avoir un cône alourdi par des entretoises inutiles à fixation de la longueur. Ceci permet d'ajuster simplement la profondeur de la chambre 12 selon la direction du regard du patient. En outre, la longueur de la distance entre le pourtour 18 de l'ouverture 16 et l'interface de fixation 20 est répétable par la ou les entretoises. L'implémentation d'entretoises permet de conserver une certaine distance fixe entre le pourtour et l'interface sans modification possible durant un test. Le réglage de la distance ne peut pas être modifié durant un test. La figure 2 montre un exemple de mode de réalisation du réglage de la distance entre le pourtour 18 de l'ouverture 16 et l'interface de fixation 20. L'utilisation d'entretoises permet aussi de refaire un même test (ou une série de mêmes tests) avec une même longueur (ou une série de mêmes longueurs) ultérieurement, car il suffit d'assembler les mêmes entretoises. Le cône 10 est donc un ensemble de modules (de sous-parties) aux dimensions connues (modules normalisés), ce qui permet de s'adapter aux patients et aux tests, le calibrage étant en outre facilité.

La figure 2 montre une vue de devant du cône de vision 10 de la figure 1 avec la paroi latérale 14 délimitant la chambre 12 entre la première extrémité 121 et la deuxième extrémité 122. Le cône de vision 10 comprend une première entretoise 221, formant la base du cône de vision. La première entretoise 221 supporte le pourtour 18 de l'ouverture 16 à la première extrémité 121 du cône de vision d'un côté. Dans un mode de réalisation, qui n'est pas couvert par les revendications, selon lequel le cône de vision 10 comprend une seule entretoise 221, cette entretoise 221 supporte alors aussi l'interface de fixation 20 de l'unité de calcul à la deuxième extrémité 122. La distance entre le pourtour 18 de l'ouverture 16 et l'interface de fixation 20 est réglée à la longueur fixe minimale. La profondeur de la chambre 12 dans la direction du regard est au minimum. Il en va de même pour la longueur de la paroi 14 entre la première extrémité 121 et la deuxième extrémité 122.

Le cône de vision 10 comprend la première entretoise 221 supportant le pourtour 18 de l'ouverture 16 à la première extrémité 121 et une ou plusieurs entretoises supplémentaires assemblées les unes aux autres, la dernière entretoise supportant l'interface de fixation 20 de l'unité de calcul à la deuxième extrémité 122. La deuxième extrémité 122 et l'interface de fixation 20 sont alors décalées de la première entretoise 221 à la dernière entretoise assemblée. Ainsi, la distance entre le pourtour 18 de l'ouverture 16 et l'interface de fixation 20 est réglée à une autre longueur fixe selon le nombre d'entretoises supplémentaires ajoutées à la première entretoise 221. La figure 2 montre un exemple du cône de vision 10 dans lequel une entretoise 222 supplémentaire est assemblée à la première entretoise 221 - la deuxième entretoise 222 supportant l'interface de fixation 20 de l'unité de calcul à la deuxième extrémité 122. La paroi 14 est allongée dans la direction du regard de la longueur de toutes entretoises supplémentaires, d'une deuxième entretoise 222 sur la figure 2 à titre d'exemple. Les entretoises sont assemblés les unes aux autres et dans le cône par leur extrémités, et ce de manière simple et sans moyen de fixation encombrant. Ainsi, avec un jeu d'entretoises de longueurs différentes, on peut obtenir différentes distances de longueur fixe.

Ainsi, la deuxième extrémité 122 et l'interface de fixation 20 sont supportées par la dernière entretoise, la N^{ième} entretoise si N entretoises supplémentaires sont assemblées les unes aux autres et à la première entretoise. La distance entre le pourtour 18 de l'ouverture 16 et l'interface de fixation 20 est réglable depuis une longueur minimale fixe lorsqu'une seule entretoise est utilisée à une longueur fixe plus grande lorsque des entretoises supplémentaires sont assemblées. La profondeur de la chambre 12 dans la direction du regard varie en fonction du nombre d'entretoise(s) utilisée(s). Les dimensions de ces entretoises étant connue, les séries de mesures sont faites dans un environnement normalisé, prédéterminé, aux dimensionnements connues. Cet environnement est pris en compte dans la calibration et tests.

Les entretoises supplémentaires sont fixées les unes aux autres de manière simple par emboîtement et/ou clipsage. Ceci peut être fait par le praticien mais également par le patient lui-même. Chaque entretoise supplémentaire se fixe à ce qui aurait été l'interface de fixation 20 de l'unité de calcul si l'entretoise supplémentaire n'avait pas été utilisée.

Les entretoises 221, 222,... forment chacune une longueur de paroi latérale 14. La profondeur des entretoises dans la direction du regard peut varier d'une entretoise à l'autre ou être similaire. Ainsi, plusieurs entretoises permettent le réglage de la distance à une longueur fixe, de par le choix de la profondeur de la ou des entretoises. Ceci permet de régler plus finement la distance entre le pourtour 18 de l'ouverture 16 et l'interface de fixation 20. Par exemple, les entretoises forment entre 5 et 15 cm de paroi latérale 14 dans la direction du regard. La première entretoise 221, formant la base du cône de vision 10, peut former 15 cm de paroi latérale 14 - cette longueur peut correspondre à la longueur de référence de la distance entre le pourtour 18 et l'interface de fixation 20 prise au centre du pourtour 18, dans la zone du pourtour 18 qui s'applique au milieu du front du patient, au droit de son nez. Les autres entretoises peuvent former entre 5 cm et 10 cm de paroi latérale 14.

La possibilité de régler la distance entre le pourtour 18 de l'ouverture 16 et l'interface de fixation 20 à une longueur fixe rend le cône peu encombrant car il n'est pas nécessaire d'avoir autant de cônes de vision que de distances souhaitées ; le cône de vision 10 est aisément transportable et tient ainsi dans une mallette. Il suffit de se munir, de transporter un jeu d'entretoises permettant de régler la distance.

Le cône de vision 10 peut comprendre en outre un ou des orifices 24 traversants dans la paroi latérale 14, les orifices 24 étant adaptés à l'envoi de stimuli visuels en différentes positions dans la chambre 12. Les orifices 24 sont visibles sur les figures 1 et 2. Ceci permet de varier le contenu des tests et d'obtenir différentes mesures dans des mises en situation différentes. Le nombre d'orifices 24 n'est pas limité. Ainsi, des LED peuvent être fixées à la paroi latérales 14, à l'extérieur de la chambre 12 de sorte à éclairer l'intérieur de la chambre 12. La lumière des LED est dans des longueurs d'ondes visibles et permet de faire des tests de convergence des yeux. Les orifices 24 peuvent être en de multiples positions - par exemple sur au moins l'une des faces supérieure, inférieure et latérales de la paroi latérale 14 - afin de créer des cibles dans la chambre 12 à différents plans de profondeur. Les orifices 24 peuvent être alignés entre la première extrémité 121 et la deuxième extrémité 122 ; ceci permet de provoquer des stimuli visuels selon différents éloignements du visage du patient. Selon les figures 1 et 2, un ou plusieurs orifices 24 peuvent être positionnés dans la paroi latérale 14, sur la face supérieure ou inférieure de la paroi latérale 14 selon une droite entre la zone du pourtour 18 qui s'applique au milieu du front du patient, au droit de son nez, et l'interface de fixation 20 dans la direction du regard. Selon la figure 2, un orifice 24 est sur l'une des faces latérales de la paroi latérale 14. Il est aussi envisageable d'ajouter d'autres signaux de stimuli tels que des sons.

Le cône de vision peut être portable sur le visage du patient. Ceci permet une grande souplesse d'utilisation pour tester tout patient dans toute condition. En particulier, pour des patients difficiles à maintenir en place lors des mesures, tels que des enfants ou bébés, la portabilité du cône de vision 10 facilite la prise de mesures. Le cône de vision est léger, ce qui facilité sa portabilité. Le cône de vision 10 peut comprendre des organes de fixation du cône à la tête du patient et/ou des poignées. Les organes de fixation, non visibles sur les figures sont par exemple une sangle réglable et/ou élastique s'étendant depuis la première extrémité 121. La sangle passe derrière et/ou par-dessus la tête du patient et est réglée de sorte que le cône de vision 10 tienne en position sur son visage. Des poignées permettent au patient de tenir le cône de vision contre son visage. Des poignées 26 sur les faces latérales de la paroi latérale 14 sont visibles sur les figures 1 et 2.

Le cône de vision 10 peut comprendre un cloisonnement de la vision de chaque œil et/ou un obturateur de la vision d'un des yeux. Ceci permet d'effectuer différents tests durant les prises de mesures. Pour obtenir le cloisonnement de la vision de chaque œil, il est possible de disposer une cloison dans la chambre 12 séparant la chambre en deux champs de vision, un pour chaque œil. Par exemple, une cloison peut être glissée depuis l'extrémité 122 dans des rainures le long de la surface interne des faces supérieure et inférieure de la paroi latérale 14 selon la direction du regard, jusqu'à l'ouverture 16 contre le visage du patient. Un obturateur peut aussi être disposé en travers de l'ouverture 16. L'obturateur peut être glissé dans une fente du cône de vision, transversale au regard, pour se mettre en travers de l'ouverture 16. L'obturateur peut comporter une zone obturée pour un œil et une zone ouverte pour la vision de l'autre œil ; on peut changer l'obturateur ou l'intervertir pour obturer l'un ou l'autre des yeux. L'obturateur peut aussi comprendre un écran amovible obturant sélectivement la vision d'un œil ou l'autre. L'écran peut être retiré ou déplacé transversalement au regard pour obturer la vision d'un œil et libérer la vision de l'autre œil.

Le cône de vision 10 peut aussi comprendre un filtre devant chaque œil pour sélectionner une partie différente sur chaque œil du stimulus visuel présenté sur l'unité de calcul. Ceci permet de varier les tests durant les prises de mesure. Il peut s'agir de filtres rouge/vert, jaune/bleue ou polarisants afin de reproduire le principe de 3-dimensions.

Le pourtour 18 de l'ouverture 16 peut être adaptable au patient, en particulier au contour du visage du patient, au contour des yeux du patient et/ou à la présence de lunettes sur le visage du patient. Ceci permet d'éviter la multiplication des tailles du cône de vision tout en s'adaptant au patient. Pour cela, le cône comporte un adaptateur qui se fixe de manière amovible à la première entretoise 221 (par exemple par clipsage). L'adaptateur supporte le pourtour 18 et l'ouverture 16 à l'extrémité 121 du cône de vision. L'adaptateur a une dimension également normalisée, au même titre que les entretoises. L'adaptateur comporte une face fixée à l'entretoise 221 et une face présentant une concavité permettant de s'adapter à la morphologie du patient - enfant, adulte, visage large, fin, présence ou non de lunettes, etc.. L'avantage est qu'il suffit d'avoir une série d'adaptateurs pour s'adapter à tout patient sans pour autant multiplier la possession de formats d'entretoises 221 - ce qui réduit l'encombrement et le coût du cône de vision.

Le cône de vision 10 peut comprendre en outre un cache 28 amovible adapté à obturer l'ouverture 16, le cache 28 comprenant un motif 30 de calibration de caméra sur sa face tournée vers l'intérieur de la chambre 12. Ceci permet une calibration aisée et rapide de caméra de l'unité de calcul. Une telle calibration peut être mise en œuvre par un praticien en charge des mesures ou par le patient lui-même. La calibration peut être mise en œuvre aisément et rapidement pour chaque distance entre le pourtour 18 de l'ouverture 16 et l'interface de fixation 20 réglée à une longueur fixe. En outre, ceci permet la calibration de caméra indépendamment du patient. Habituellement, le calibrage de caméra dépend du patient ; ce dernier doit fixer des points sur un écran et la position des yeux sert de référence pour la calibration des caméras. Or il peut arriver que le patient ne parvienne pas à procéder à cette opération car il ne la comprend pas ou parce que ses yeux sont constamment en mouvement. Le motif 30 sur le cache 28 permet dans toutes ces circonstances de procéder au calibrage de caméra.

Le fait que la distance entre le pourtour 18 de l'ouverture 16 et l'interface de fixation 20 soit réglable à une longueur fixe permet de réaliser la calibration dans un environnement normalisé, prédéterminé, aux dimensionnements connues. Cet environnement est pris en compte dans la calibration et tests. L'environnement est normalisé dans le temps et même d'un patient à un autre. Par ailleurs, le fait que le réglage à une longueur fixe soit obtenu par une ou plusieurs entretoises formant la paroi et ayant une dimension connue et normalisée permet de faciliter et améliorer la calibration.

Egalement, le motif 30, et possiblement le cache 28, est de préférence bombé. Il peut être bombé vers l'intérieur ou l'extérieur de la chambre 12, ou en d'autres termes, concave ou convexe par rapport à la chambre 12. Le motif 30 n'est pas plat. Ceci permet de calibrer la caméra avec un motif en trois dimensions, présentant une profondeur. On parvient ainsi à faciliter et améliorer la calibration de la caméra. En effet, avec un motif de calibration plan, il n'est pas possible de résoudre les équations de calibrage ; plusieurs images de ce même plan doivent alors être prises dans différentes orientations pour y parvenir, ce qui complexifie la calibration - voire la rend de mauvaise qualité ou impossible si le calibrage est réalisé par le patient. Le motif 30 bombé évite de tels problèmes.

Le cache 28 obturant l'ouverture 16, le motif 30 est alors positionné précisément sur le cône 10 (à même le cône) afin de garantir son positionnement absolu vis à vis de la position des caméras. Ceci permet d'avoir une référence absolue de chacun des points du motif 30. Ceci permet de donner à l'algorithme de calibration une position et une orientation absolue. Par exemple, le motif est positionné précisément sur le cône via des pions de centrage.

De plus, le cache 28 obturant l'ouverture 16, le motif 30 est alors positionné au même endroit que les yeux du patient. Ceci permet d'extraire immédiatement la distance entre les deux pupilles (ce qui peut être utile, notamment en ophtalmologie). Cela permet aussi d'extraire le centre des yeux à chaque instant. Comme la calibration est effectuée là où le patient regarde sur l'unité de calcul, on obtient une estimée de l'orientation de chaque œil vis à vis d'un référentiel fixé à la tête.

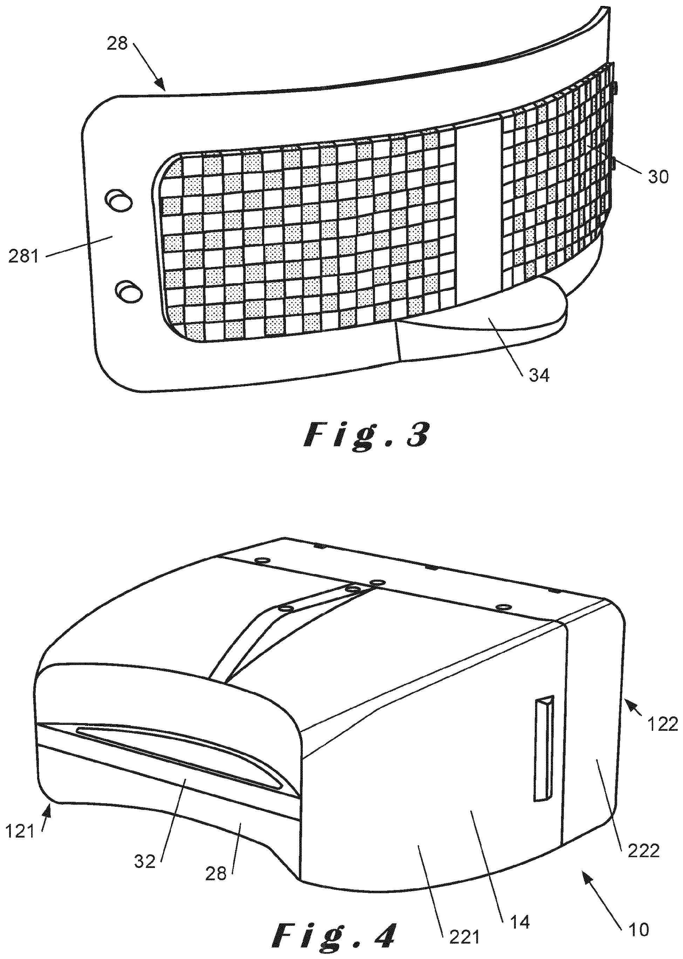

La figure 3 montre le cache 28. La taille du cache 28 est telle que le cache 28 obture l'ouverture 16 de sorte à complètement assombrir la chambre 12. La forme du cache 28 s'adapte à celle du pourtour 18. Le cache 28 peut être plan (avec possiblement le motif 30 bombé (vers l'intérieur ou l'extérieur de la chambre 12, ou en d'autres termes, concave ou convexe par rapport à la chambre 12) sur la face 281 du cache) si le pourtour 18 et l'ouverture 16 le sont. Le cache 28 est aussi de préférence bombé (vers l'intérieur ou l'extérieur de la chambre 12, ou en d'autres termes, concave ou convexe par rapport à la chambre 12) pour s'adapter à la concavité du pourtour 18, le cas échéant. Le motif 30 (et possiblement le cache 28) est de préférence concave, bombé vers l'intérieur de la chambre 12. Le motif 30 est sur la face 281 du cache 28 destinée à être tournée vers l'intérieur de la chambre 12. La figure 4 montre le cache 28 en position sur le cône de vision 10 - la première entretoise 221 en particulier. La face 281 est tournée vers la chambre 12 et le cache 28 est à la place des yeux du patient ; ceci permet un calibrage de caméra sans que le cône de vision soit en position sur le patient. Le motif est par exemple un damier, un quadrillage noir et blanc formant des pixels correspondant à une grandeur physique connue. La distance entre le pourtour 18 et l'interface de fixation 20 de l'unité de calcul étant réglée à une longueur fixe, la distance entre les yeux et les caméras est aussi connue. Ceci permet le calibrage de caméra de sorte à convenablement détecter le regard du patient par l'unité de calcul. Le cache 28 peut comporter une poignée 32 sur sa face tournée à l'opposé de la chambre 12 ; une telle poignée facilite la mise en position et le retrait du cache 28. En outre, le cache 28 peut comprendre une aspérité 34 sur la face tournée vers l'intérieur de la chambre 12. L'aspérité 34 permet de colmater la cavité 19 du pourtour 18 adaptant le cône de vision au nez du patient. L'aspérité 34 empêche la lumière de pénétrer dans la chambre 12 durant la phase de calibration de caméra.

Le cône de vision est un ensemble passif. Autrement dit, la structure du cône de vision est apte à fonctionner sans alimentation électrique, indépendamment d'une alimentation électrique. Le cône de vision est par exemple en plastique, biocompatible, de qualité médicale. Le cône de vision 10 peut être désinfecté ou stérilisé entre deux patients. Le cône de vision est dépourvu de recoins difficiles ou impossible à stériliser. Le cône de vision est en outre résistant et peut chuter d'une hauteur de 1 mètre.

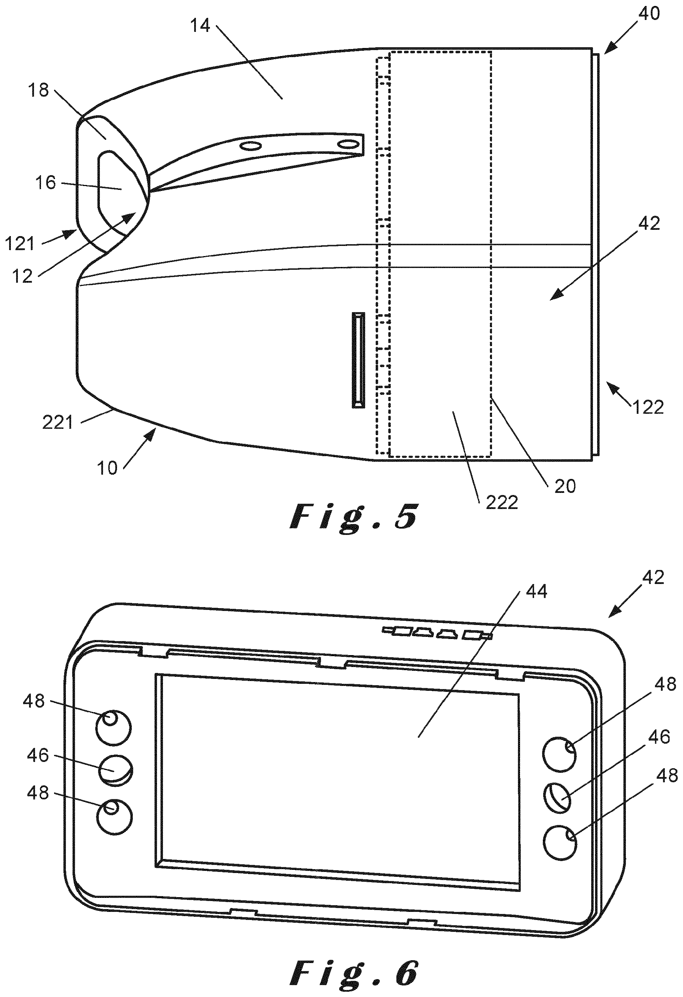

L'invention se rapporte aussi à un dispositif 40 de détection du mouvement des yeux d'un patient. Selon un exemple représenté sur la figure 5, le dispositif 40 comprend le cône de vision 10 et une unité de calcul 42 fixée à l'interface de fixation 20 du cône de vision. L'unité de calcul 42 comprend des caméras de suivi du mouvement des yeux et un écran d'affichage de stimuli visuels. La résolution des caméras est par exemple de 640/480 pixel. La figure 6 montre un exemple de réalisation de l'unité de calcul 42. L'écran 44 occupe une large partie d'un plan destiné à être à l'extrémité 122 du cône de vision. Par exemple, un écran de 15 cm de diagonal permet des tests à +/- 27° de variation horizontale du regard et +/- 16° de variation verticale du regard. De part et d'autre de l'écran 44, des ouvertures sont destinées à recevoir chacune une caméra 46 pour détecter le mouvement des yeux du patient ; la présence de deux caméras permet de focaliser une caméra sur chaque œil, augmentant ainsi la résolution spatiale. Les caméras 46 sont de préférence à mi-hauteur de part et d'autre de l'écran 44 et donc à la hauteur des yeux ; ceci présente l'avantage de mieux détecter le mouvement des yeux sans retraitement des données pour corriger un possible angle avec le plan contenant les yeux. En outre, l'unité de calcul 42 peut recevoir des émetteurs 48. Il s'agit de préférence d'émetteurs dans la longueur de l'infrarouge (par exemple 940 nm), non visible par l'œil humain mais permettant d'illuminer les yeux pour la détection du mouvement des yeux par les caméras 46. L'unité de calcul 42 et donc le dispositif 40 permettent aussi de facilement changer l'écran, les caméras et les émetteurs pour en augmenter la version.

Selon la figure 5, la distance entre le pourtour 18 de l'ouverture 16 et l'interface de fixation 20 de l'unité de calcul 42 est réglable à une longueur fixe par les entretoises 221, 222, ... formant la paroi latérale 14. On peut ainsi faire varier la distance entre le visage et l'écran 44 de l'unité de calcul 42 diffusant les stimuli visuels. A titre d'exemple, le cône de vision 10 comprend la première entretoise 221 avec l'ouverture 16 à la première extrémité 121 et comprend la deuxième entretoise 222 assemblée à la première entretoise 221. La deuxième extrémité 122 avec l'interface de fixation 20 sont supportées par la deuxième entretoise 222. L'unité de calcul 42 est fixée à l'interface de fixation 20. Il est envisageable d'avoir deux entretoises ou plus, les entretoises étant assemblées les unes aux autres. L'unité de calcul 42 est fixé à l'interface de fixation 20 supportée par la dernière entretoise.

Le dispositif 40 peut être portable comme décrit ci-dessus en référence au cône de vision 10 (en fixant le cône sur la tête du patient) ou peut aussi comprendre un support supportant l'unité de calcul, le cône de vision étant fixé à l'unité de calcul par l'interface de fixation 20. Il est ainsi possible de positionner l'unité de calcul de manière immobile. Ceci permet aussi d'alléger le dispositif 40. Le visage du patient reste alors dans la même position - ce qui est aussi un avantage si le patient a tendance à trop bouger. Une sangle décrite précédemment permet d'immobiliser le visage du patient contre le pourtour 18. Le support peut être un pied que l'on pose au sol ou sur une table ; le support peut être aussi un bras articulé, fixé à un banc de mesures par exemple.

L'unité de calcul 42 peut être reliée à un ordinateur du praticien pour sauvegarder les mesures durant les séries de mesures ou même après. Que le dispositif 40 soit portable ou supporté par un support via l'unité de calcul 42, la liaison à l'ordinateur peut être faite à distance ou par une liaison filaire. Le dispositif 40 est ainsi polyvalent.

L'invention se rapporte aussi à une méthode de détection du mouvement des yeux d'un patient. La méthode comprend une étape de fourniture du dispositif 40 tel que décrit précédemment et une étape de réglage de la distance entre le pourtour 18 de l'ouverture 16 et l'interface de fixation 20 à une longueur fixe. On détermine ainsi la profondeur de la chambre 12 dans la direction du regard, entre le visage du patient et l'écran 44 de l'unité de calcul 42. Ceci permet de réaliser aisément plusieurs séries de mesures en faisant varier la distance entre le visage et l'écran 44.

Afin de régler la distance entre le pourtour 18 et l'interface de fixation 20 - et donc la distance entre le visage et l'écran - la méthode comprend une étape dans laquelle on utilise, intercale, ajoute plusieurs entretoises 221, 222,... pour former la paroi latérale 14. La distance est facilement réglable puis demeure fixe durant la série de mesures de sorte à faire des mesures dans un environnement normalisé.

Avant de lancer une série de mesures, la méthode peut en outre comprendre une étape de calibration de caméra. Au cours de cette étape, l'ouverture 16 est obturée par le cache 28 amovible comprenant le motif de calibration 30 de caméra sur sa face tournée vers l'intérieur de la chambre 12. Ainsi, il est aisé pour le praticien ou le patient lui-même d'effectuer la calibration une fois la distance entre le pourtour 18 et l'interface de fixation 20 réglée à une longueur fixe. L'environnement défini par le cône de vision étant normalisé, prédéterminé, aux dimensionnements connues, ceci permet de facilement mener la calibration et les tests. Il est aussi aisé et rapide de faire une nouvelle calibration après modification de la distance ; plusieurs séries de mesures peuvent être réalisées sans perdre de temps dans la calibration de caméra. La calibration étant aisée, le patient peut réaliser les tests chez lui sans la présence de praticien.

La méthode peut aussi comprendre en outre une étape de stimulation des yeux par l'envoi de stimuli visuels dans la chambre 12 par les orifices 24 traversants de la paroi latérale 14.

La méthode de détection du mouvement des yeux d'un patient est donc rapide à mettre en place car le réglage de la distance entre les yeux et l'écran, la calibration des caméras, la mise en service par allumage de l'unité de calcul et l'éventuelle connexion à un ordinateur du praticien sont rapides.

La présente invention a été décrite en relation avec des modes de réalisations spécifiques, qui ont une valeur purement illustrative et ne doivent pas être considérés comme limitatifs. D'une manière générale, il apparaîtra évident pour un homme du métier que la présente invention n'est pas limitée aux exemples illustrés et/ou décrits ci-dessus, l'invention étant définie par les revendications annexées.

## Revendications

1. Cône de vision (10) pour la détection du mouvement des yeux d'un patient, le cône de vision (10) comprenant
• Une chambre (12) avec une première extrémité (121) et une deuxième extrémité (122),
• Une paroi latérale (14) délimitant la chambre (12) entre la première extrémité et la deuxième extrémité,
• Un pourtour (18) d'une ouverture (16) apte à être en contact du front du patient à la première extrémité (121),
• Une interface de fixation (20) d'une unité de calcul à la deuxième extrémité (122),
• Une première entretoise (221) supportant le pourtour (18) de l'ouverture (16) et une ou plusieurs entretoises supplémentaires (222,...) amovibles assemblées les unes aux autres formant la paroi latérale (14),
dans lequel
la distance entre le pourtour (18) de l'ouverture (16) et l'interface de fixation (20) étant réglable, à une longueur fixe par une ou plusieurs desdites entretoises supplémentaires (222,...) amovibles formant la paroi latérale (14).

2. Cône de vision (10) selon la revendication 1, dans lequel la dernière entretoise supporte l'interface de fixation (20) de l'unité de calcul à la deuxième extrémité (122).

3. Cône de vision (10) selon l'une des revendications 1 à 2, dans lequel les entretoises (221, 222,...) forment entre 5 et 15 cm de paroi latérale (14).

4. Cône de vision (10) selon l'une des revendications précédentes, comprenant en outre un ou des orifices (24) traversants dans la paroi latérale (14), les orifices (24) étant adaptés à l'envoi de stimuli visuels en différentes position dans la chambre (12).

5. Cône de vision (10) selon l'une des revendications précédentes, **caractérisé en ce que** le cône de vision (10) est portable sur le visage du patient.

6. Cône de vision (10) selon l'une des revendications précédentes, comprenant un cloisonnement de la vision de chaque œil et/ou un obturateur de la vision d'un des yeux.

7. Cône de vision (10) selon l'une des revendications précédentes, comprenant en outre un cache (28) amovible adapté à obturer l'ouverture (16), le cache (28) comprenant un motif (30) de calibration de caméra sur sa face tournée vers l'intérieur de la chambre (12).

8. Cône de vision (10) selon la revendication précédente, dans lequel le motif (30) est concave.

9. Dispositif (40) de détection du mouvement des yeux d'un patient comprenant
• le cône de vision (10) selon l'une des revendications précédentes et
• une unité de calcul (42) fixée à l'interface de fixation (20) du cône de vision (10), l'unité de calcul (42) comprenant des caméras (46) de suivi du mouvement des yeux et un écran (44) d'affichage de stimuli visuels.

10. Dispositif (40) selon la revendication précédente, dans lequel le cône de vision (10) comprend un cache (28) amovible adapté à obturer l'ouverture (16), le cache comprenant un motif (30) de calibration des caméras (46) sur sa face tournée vers l'intérieur de la chambre (12).

11. Dispositif (40) selon l'une des revendications 9 ou 10, comprenant en outre un support supportant l'unité de calcul (42), le cône de vision étant fixé à l'unité de calcul par l'interface de fixation (20).

12. Méthode de détection du mouvement des yeux d'un patient, comprenant les étapes de
• Fourniture d'un dispositif (40) de détection du mouvement des yeux d'un patient comprenant
∘ Un cône de vision (10) selon la revendication 1,
∘ Une unité de calcul (42) fixée à l'interface de fixation (20) du cône de vision (10), l'unité de calcul (42) comprenant des caméras (46) de suivi du mouvement des yeux et un écran (44) d'affichage de stimuli visuels
• Réglage de la distance entre le pourtour (18) de l'ouverture (16) et l'interface de fixation (20) à une longueur fixe d'une valeur souhaitée, par plusieurs entretoises (221, 222,...) qui sont utilisées pour former la paroi latérale (14),
l'étape de réglage à une longueur fixe étant effectuée par ajout d'une ou plusieurs entretoises supplémentaires, le nombre d'entretoise(s) nécessaire(s) étant ajusté à la longueur souhaitée.

13. Méthode selon la revendication 12, comprenant en outre une étape de calibration des caméras (46) au cours de laquelle l'ouverture (16) est obturée par un cache (28) amovible comprenant un motif de calibration (30) de caméra sur sa face tournée vers l'intérieur de la chambre (12).

14. Méthode selon la revendication 13, dans laquelle le motif de calibration (30) est concave.

## Patentansprüche

1. Sehkegel (10) zum Erkennen der Bewegung der Augen eines Patienten, wobei der Sehkegel (10) umfasst
- eine Kammer (12) mit einem ersten Ende (121) und einem zweiten Ende (122),
- eine Seitenwand (14), die die Kammer (12) zwischen dem ersten Ende und dem zweiten Ende begrenzt,
- einen Rand (18) einer Öffnung (16), der geeignet ist, mit der Stirn des Patienten in Kontakt zu gelangen, am ersten Ende (121),
- eine Befestigungsschnittstelle (20) für eine Recheneinheit am zweiten Ende (122),
- einen ersten Abstandshalter (221), der den Rand (18) der Öffnung (16) trägt, und einen oder mehrere abnehmbare zusätzliche Abstandshalter (222, ...), die zusammengebaut sind, wodurch sie die Seitenwand (14) bilden,
wobei der Abstand zwischen dem Rand (18) der Öffnung (16) und der Befestigungsschnittstelle (20) über einen oder mehrere der abnehmbaren zusätzlichen Abstandshalter (222, ...), die die Seitenwand (14) bilden, auf eine feste Länge eingestellt werden kann.

2. Sehkegel (10) nach Anspruch 1, wobei der letzte Abstandshalter die Befestigungsschnittstelle (20) der Recheneinheit am zweiten Ende (122) trägt.

3. Sehkegel (10) nach einem der Ansprüche 1 bis 2, wobei die Abstandshalter (221, 222, ...) zwischen 5 und 15 cm der Seitenwand (14) bilden.

4. Sehkegel (10) nach einem der vorstehenden Ansprüche, der weiter eine oder mehrere Durchgangsöffnungen (24) in der Seitenwand (14) umfasst, wobei die Öffnungen (24) zum Senden von visuellen Reizen an unterschiedlichen Positionen in der Kammer (12) ausgelegt sind.

5. Sehkegel (10) nach einem der vorstehenden Ansprüche, **dadurch gekennzeichnet, dass** der Sehkegel (10) auf dem Gesicht des Patienten getragen werden kann.

6. Sehkegel (10) nach einem der vorstehenden Ansprüche, der eine Abschottung für das Sehfeld jedes Auges und/oder einen Verschluss für das Sehfeld eines der Augen umfasst.

7. Sehkegel (10) nach einem der vorstehenden Ansprüche, der weiter eine abnehmbare Abdeckung (28) umfasst, die zum Verschließen der Öffnung (16) ausgelegt ist, wobei die Abdeckung (28) auf ihrer dem Inneren der Kammer (12) zugewandten Seite ein Kamerakalibrierungsmuster (30) umfasst.

8. Sehkegel (10) nach dem vorstehenden Anspruch, wobei das Muster (30) konkav ist.

9. Vorrichtung (40) zum Erkennen der Bewegung der Augen eines Patienten, umfassend
- den Sehkegel (10) nach einem der vorstehenden Ansprüche, und
- eine Recheneinheit (42), die an der Befestigungsschnittstelle (20) des Sehkegels (10) befestigt ist, wobei die Recheneinheit (42) Kameras (46) zum Verfolgen der Bewegung der Augen und einen Bildschirm (44) zum Anzeigen von visuellen Reizen umfasst.

10. Vorrichtung (40) nach dem vorstehenden Anspruch, wobei der Sehkegel (10) eine abnehmbare Abdeckung (28) umfasst, die zum Verschließen der Öffnung (16) ausgelegt ist, wobei die Abdeckung auf ihrer dem Inneren der Kammer (12) zugewandten Seite ein Kalibrierungsmuster (30) für die Kameras (46) umfasst.

11. Vorrichtung (40) nach einem der Ansprüche 9 oder 10, die weiter einen Träger umfasst, der die Recheneinheit (42) trägt, wobei der Sehkegel über die Befestigungsschnittstelle (20) an der Recheneinheit befestigt ist.

12. Verfahren zum Erkennen der Bewegung der Augen eines Patienten, das die folgenden Schritte umfasst
- Bereitstellen einer Vorrichtung (40) zum Erkennen der Bewegung der Augen eines Patienten, umfassend
- einen Sehkegel (10) nach Anspruch 1,
- eine Recheneinheit (42), die an der Befestigungsschnittstelle (20) des Sehkegels (10) befestigt ist, wobei die Recheneinheit (42) Kameras (46) zum Verfolgen der Bewegung der Augen und einen Bildschirm (44) zum Anzeigen von visuellen Reizen umfasst,
- Einstellen des Abstands zwischen dem Rand (18) der Öffnung (16) und der Befestigungsschnittstelle (20) auf eine feste Länge mit einem gewünschten Wert über mehrere Abstandshalter (221, 222, ...), die verwendet werden, um die Seitenwand (14) zu bilden,
wobei der Schritt des Einstellens auf eine feste Länge durch Hinzufügen eines oder mehrerer zusätzlicher Abstandshalter erfolgt, wobei die Anzahl des/der erforderlichen Abstandshalter(s) auf die gewünschte Länge angepasst wird.

13. Verfahren nach Anspruch 12, das weiter einen Schritt des Kalibrierens der Kameras (46) umfasst, in dessen Verlauf die Öffnung (16) über eine abnehmbare Abdeckung (28), die auf ihrer dem Inneren der Kammer (12) zugewandten Seite ein Kamerakalibrierungsmuster (30) umfasst, verschlossen wird.

14. Verfahren nach Anspruch 13, wobei das Kalibrierungsmuster (30) konkav ist.

## Claims

1. A viewing cone (10) for detecting the movement of the eyes of a patient, the viewing cone (10) comprising
- A chamber (12) with a first end (121) and a second end (122),
- A lateral wall (14) delimiting the chamber (12) between the first end and the second end,
- A periphery (18) of an opening (16) intended to be in contact with the forehead of the patient at the first end (121),
- An attachment interface (20) for attaching a processing unit at the second end (122),
- A first spacer (221) supporting the periphery (18) of the opening (16) and one or more additional removable spacers (222, etc.) joined together forming the lateral wall (14)
wherein
the distance between the periphery (18) of the opening (16) and the attachment interface (20) is adjustable, to a fixed length by one or more of said removable spacers (222, etc.) forming the lateral wall (14).

2. The viewing cone (10) according to claim 1, wherein the last spacer supports the attachment interface (20) for attaching the processing unit at the second end (122).

3. The viewing cone (10) according to one of claims 1 to 2, wherein the spacers (221, 222, etc.) form between 5 and 15 cm of lateral wall (14).

4. The viewing cone (10) according to any of the preceding claims, further comprising one or more through-orifices (24) in the lateral wall (14), the orifices (24) being adapted to send visual stimuli to different positions in the chamber (12).

5. The viewing cone (10) according to one of the preceding claims, **characterised in that** the viewing cone (10) is wearable on the face of the patient.

6. The viewing cone (10) according to one of the preceding claims, comprising a partition for the vision of each eye and/or a shutter for the vision of one of the eyes.

7. The viewing cone (10) according to one of the preceding claims, further comprising a removable cover (28) adapted to obturate the opening (16), the cover (28) comprising a camera calibration pattern (30) on its side turned towards the inside of the chamber (12).

8. The viewing cone (10) according to the preceding claim, wherein the pattern (30) is concave.

9. A device (40) for detecting the movement of the eyes of a patient, comprising
- the viewing cone (10) according to one of the preceding claims and
- a processing unit (42) attached to the attachment interface (20) of the viewing cone (10), the processing unit (42) comprising cameras (46) for tracking the movement of the eyes and a screen (44) for displaying visual stimuli.

10. The device (40) according to the preceding claim, wherein the viewing cone (10) comprises a removable cover (28) adapted to obturate the opening (16), the cover comprising a pattern (30) for calibrating the cameras (46) on its side turned towards the inside of the chamber (12).

11. The device (40) according to one of claims 9 or 10, further comprising a support supporting the processing unit (42), the viewing cone being attached to the processing unit by the attachment interface (20).

12. A method for detecting the movement of the eyes in a patient, comprising the steps of
- Supplying a device (40) for detecting the movement of the eyes of a patient comprising
∘ A viewing cone (10) according to claim 1
∘ A processing unit (42) attached to the attachment interface (20) of the viewing cone (10), the processing unit (42) comprising cameras (46) for tracking the movement of the eyes and a screen (44) for displaying visual stimuli
- Setting the distance between the periphery (18) of the opening (16) and the attachment interface (20) to a fixed length of a desired value by a plurality of spacers (221, 222, etc.) used to form the lateral wall (14),
the setting step to a fixed length being performed by addition of one or more additional spacers, the necessary number of spacers being adjusted to the desired length.

13. The method according to claim 12, further comprising a step of calibrating the cameras (46) during which the opening (16) is obturated by a removable cover (28) comprising a camera calibration pattern (30) on its side turned towards the inside of the chamber (12).

14. The method of claim 13, wherein the calibration pattern (30) is concave.
